# EUROPEAN PATENT APPLICATION

(11) **EP 3 939 955 A1**
(43) Date of publication of application: **19.01.2022**
(21) Application number: 20769363.1
(22) Date of filing: 11.03.2020
(51) Int. Cl.: C07C 19/08, C07C 17/383, C09K 5/04

(54) **COMPOSITION CONTAINING 1,1,2-TRIFLUOROETHANE**

(30) Priority: 11.03.2019 JP 2019044201
(71) Applicant: DAIKIN INDUSTRIES, LTD., Osaka-shi, Osaka 530-8323 (JP)
(72) Inventor: TAKAHASHI, Kazuhiro, Osaka-shi, Osaka 530-8323 (JP)
(74) Representative: Hoffmann Eitle
(86) International application number: PCT/JP2020/010656
(87) International publication number: WO 2020/184635

(57) **Abstract**

Provided is a novel composition containing HFC-143. The composition contains HFC-143 and at least one additional compound selected from the group consisting of HFO-1132a, HFO-1123, HFC-143a, CTFE, HCFO-1131a, HFC-152a, HFC-152, HCFO-1131(E), HCFO-1131(Z), HFC-134a, HFC-161, HCFC-141, HCFC-123, HCFC-142, HCFC-142a, HCFO-1122, HCFO-1122a, HFO-1141, HCFC-21, HCFC-22, HFC-23, ethylene, and acetylene.

## Description

### Technical Field

The present disclosure relates to a composition containing 1,1,2-trifluoroethane (HFC-143).

### Background Art

1,1,2-Trifluoroethane (HFC-143) is useful in expansion agents for polyolefins and polyurethanes, aerosol propellants, refrigerants, heat transfer media, gaseous dielectrics, fire extinguishing agents, power cycle working fluids, polymerization media, particulate removal fluids, carrier fluids, buffing abrasive agents, and displacement drying agents (PTL 1). HFC-143 has a boiling point of about 4°C.

### Citation List

### Patent Literature

PTL 1: WO94/011460A

### Summary of Invention

### Technical Problem

An object of the present disclosure is to provide a novel composition containing HFC-143.

### Solution to Problem

### Item 1.

A composition comprising
HFC-143, and
at least one additional compound selected from the group consisting of 1,1-difluoroethylene (HFO-1132a), 1,1,2-trifluoroethylene (HFO-1123), 1,1,1-trifluoromethane (HFC-143a), 1-chloro-1,2,2-trifluoroethylene (CTFE), 1-chloro-1-fluoroethylene (HCFO-1131a), 1,1-difluoroethane (HFC-152a), 1,2-difluoroethane (HFC-152), trans-1-chloro-2-fluoroethylene (HCFO-1131(E)), cis-1-chloro-2-fluoroethylene (HCFO-1131(Z)), 1,1,1,2-tetrafluoroethane (HFC-134a), fluoroethane (HFC-161), 1,2-dichloro-1-fluoroethane (HCFC-141), 1,1-dichloro-2,2,2-trifluoroethane (HCFC-123), 1-chloro-2,2-difluoroethane (HCFC-142), 1-chloro-1,2-difluoroethane (HCFC-142a), 1-chloro-2,2-difluoroethylene (HCFO-1122), 1-chloro-1,2-difluoroethylene (HCFO-1122a), fluoroethylene (HFO-1141), dichlorofluoromethane (HCFC-21), chlorodifluoromethane (HCFC-22), trifluoromethane (HFC-23), ethylene, and acetylene.

### Item 2.

The composition according to Item 1, wherein HFC-143 and the additional compound are in the form of an azeotropic or azeotrope-like mixture thereof.

### Item 3.

The composition according to Item 1 or 2, wherein the additional compound is present in a total amount of more than 0 mass% and 30 mass% or less, based on the entire mixture of HFC-143 and the additional compound.

### Item 4.

Use of the composition of any one of Items 1 to 3 as a heat transfer medium composition.

### Item 5.

A method for separating an additional compound from HFC-143 in a mixture containing HFC-143 and the additional compound,
the additional compound being at least one compound selected from the group consisting of 1,1-difluoroethylene (HFO-1132a), 1,1,2-trifluoroethylene (HFO-1123), 1,1,1-trifluoromethane (HFC-143a), 1-chloro-1,2,2-trifluoroethylene (CTFE), 1-chloro-1-fluoroethylene (HCFO-1131a), 1,1-difluoroethane (HFC-152a), 1,2-difluoroethane (HFC-152), trans-1-chloro-2-fluoroethylene (HCFO-1131(E)), cis-1-chloro-2-fluoroethylene (HCFO-1131(Z)), 1,1,1,2-tetrafluoroethane (HFC-134a), fluoroethane (HFC-161), 1,2-dichloro-1-fluoroethane (HCFC-141), 1,1-dichloro-2,2,2-trifluoroethane (HCFC-123), 1-chloro-2,2-difluoroethane (HCFC-142), 1-chloro-1,2-difluoroethane (HCFC-142a), 1-chloro-2,2-difluoroethylene (HCFO-1122), 1-chloro-1,2-difluoroethylene (HCFO-1122a), fluoroethylene (HFO-1141), dichlorofluoromethane (HCFC-21), chlorodifluoromethane (HCFC-22), trifluoromethane (HFC-23), ethylene, and acetylene,
the method comprising
   (1) supplying a starting composition containing HFC-143 and the additional compound to a first distillation column to extract
      an azeotropic mixture composition containing HFC-143 and the additional compound as a first distillate, and
      a composition that is richer in concentration of either HFC-143 or the additional compound than the starting composition as a first distillation-column-bottom composition; and
   (2) optionally supplying the first distillate or the first distillation-column-bottom composition to a second distillation column that is different from the first distillation column in terms of operation conditions to form a composition that is richer in concentration of either HFC-143 or the additional compound than the starting composition.

### Advantageous Effects of Invention

A novel composition containing HFC-143 is provided. Brief Description of Drawings

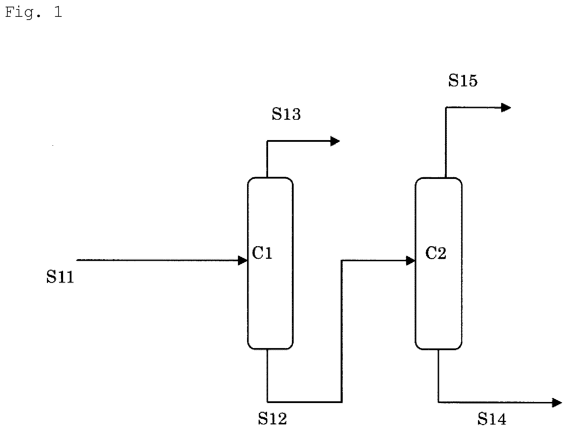

Fig. 1 is a diagram that shows the operation in an Example in which a composition containing HFC-143 and additional components such as HFO-1123, ethylene, HFC-161, HFC-152, HCFC-133, HCFC-123, and HCFC-21 is distilled by using distillation columns.

### Description of Embodiments

### Definition of Terms

In the present specification, the term "refrigerant" includes at least compounds that are specified in ISO 817 (International Organization for Standardization), and that are given a refrigerant number (ASHRAE number) representing the type of refrigerant with "R" at the beginning; and further includes refrigerants that have properties equivalent to those of such refrigerants, even though a refrigerant number is not yet given. Refrigerants are broadly divided into fluorocarbon compounds and non-fluorocarbon compounds in terms of the structure of the compounds. Fluorocarbon compounds include chlorofluorocarbons (CFC), hydrochlorofluorocarbons (HCFC), and hydrofluorocarbons (HFC). Non-fluorocarbon compounds include propane (R290), propylene (R1270), butane (R600), isobutane (R600a), carbon dioxide (R744), ammonia (R717), and the like.

In the present specification, the phrase "composition comprising a refrigerant" at least includes (1) a refrigerant itself (including a mixture of refrigerants), (2) a composition that further comprises other components and that can be mixed with at least a refrigeration oil to obtain a working fluid for a refrigerating machine, and (3) a working fluid for a refrigerating machine containing a refrigeration oil. In the present specification, of these three embodiments, the composition (2) is referred to as a "refrigerant composition" so as to distinguish it from a refrigerant itself (including a mixture of refrigerants). Further, the working fluid for a refrigerating machine (3) is referred to as a "refrigeration oil-containing working fluid" so as to distinguish it from the "refrigerant composition."

In the present specification, the term "azeotrope-like composition" refers to a composition that can be handled in substantially the same manner as azeotropic compositions. Specifically, in the present specification, the term "azeotrope-like composition" means a mixture composed of two or more substances that behave substantially as a single substance with a constant boiling point or substantially a constant boiling point. A feature of an azeotrope-like composition is that vapor generated by evaporating or distilling the composition in liquid form has a formulation substantially unchanged from the formulation of the liquid. Specifically, in the present specification, a mixture that can be boiled, distilled, or refluxed without a substantial compositional change is referred to as an "azeotrope-like composition." Specifically, a composition having a difference between the bubble-point pressure and the dew-point pressure of 3 percent or less (based on the bubble-point pressure) at a specific temperature is defined as an azeotrope-like composition in the present disclosure.

In the present specification, when the term "alternative" is used in a context in which the first refrigerant is replaced with the second refrigerant, the first type of "alternative" means that equipment designed for operation using the first refrigerant can be operated using the second refrigerant under optimum conditions, optionally with changes of only a few parts (at least one of the following: refrigeration oil, gasket, packing, expansion valve, dryer, and other parts) and equipment adjustment. In other words, this type of alternative means that the same equipment is operated with an alternative refrigerant. Embodiments of this type of "alternative" include "drop-in alternative," "nearly drop-in alternative," and "retrofit," in the order in which the extent of changes and adjustment necessary for replacing the first refrigerant with the second refrigerant is smaller.

The term "alternative" also includes a second type of "alternative," which means that equipment designed for operation using the second refrigerant is operated for the same use as the existing use with the first refrigerant by using the second refrigerant. This type of alternative means that the same use is achieved with an alternative refrigerant.

In the present specification, the term "refrigerating machine" refers to machines in general that draw heat from an object or space to make its temperature lower than the temperature of ambient air, and maintain a low temperature. In other words, refrigerating machines refer to conversion machines that gain energy from the outside to do work, and that perform energy conversion, in order to transfer heat from where the temperature is lower to where the temperature is higher.

In the present specification, GWP is calculated as specified in the IPCC Fifth Assessment Report (AR5).

### 1. Composition

### 1.1 Additional Compound

The refrigerant according to the present disclosure further contains at least one additional compound selected from the group consisting of HFO-1132a, HFO-1123, HFC-143a, CTFE, HCFO-1131a, HFC-152a, HFC-152, HCFO-1131(E), HCFO-1131(Z), HFC-134a, HFC-161, HCFC-141, HCFC-123, HCFC-142, HCFC-142a, HCFO-1122, HCFO-1122a, HFO-1141, HCFC-21, HCFC-22, HFC-23, ethylene, and acetylene, in addition to HFC-143.

The mixture of HFC-143 and all of the at least one additional compound has excellent stability. Additionally, because HFC-143 and additional compounds each can act as a refrigerant, the mixture can be used as a refrigerant, with a significantly lower ODP (ozone depletion potential) than conventionally used CFC refrigerants and HCFC refrigerants. The mixture is also useful as an intermediate for organic synthesis.

From the standpoint of stability and performance as a refrigerant or in the use as an intermediate for organic synthesis, the additional compound is present in an amount of preferably more than 0 mass% and 30 mass% or less, more preferably more than 0 mass% and 20 mass% or less, still more preferably more than 0 mass% and 10 mass% or less, and yet more preferably more than 0 mass% and 1 mass% or less, based on the entire mixture of HFC-143 and the additional compound.

### 1.2 Azeotropic or Azeotrope-Like Composition

The mixture of HFC-143 and all of the at least one additional compound is preferably an azeotropic or azeotrope-like composition. The azeotropic or azeotrope-like composition can serve as an important composition in performing azeotropic distillation for separating an additional component from HFC-143 in a mixture of HFC-143 and the additional component.

Azeotropic distillation refers to a method of concentrating or separating a target product by operating a distillation column under conditions such that an azeotropic or azeotrope-like composition is separated. In some cases, azeotropic distillation can distill only the component to be separated, but in other cases, azeotropic distillation may occur only when another component that forms an azeotropic mixture with one or more components to be separated is externally added. In a narrow sense, only the latter is called azeotropic distillation. For example, an additional component can be separated from HFC-143 by extracting an azeotropic or azeotrope-like composition containing HFC-143 and the additional component from a composition containing at least HFC-143 and the additional component by azeotropic distillation.

In order for the mixture of HFC-143 and all of the at least one additional compound to become azeotropic or azeotrope-like, the additional compound is present in an amount of preferably more than 0 mass% and 1 mass% or less, more preferably more than 0 mass% and 0.5 mass% or less, and still more preferably more than 0 mass% and 0.1 mass% or less, based on the entire mixture.

### 1.3 Heat Transfer Medium Composition

The composition according to the present disclosure can be used as a heat transfer medium composition.

The composition according to the present disclosure used as a heat transfer medium composition may further comprise at least one other component in addition to the additional compound. The composition according to the present disclosure can be further used to obtain a working fluid for a refrigerating machine by mixing the composition with at least a refrigeration oil (the composition according to the present disclosure in this case being referred to as "the refrigerant composition according to the present disclosure").

The refrigerant composition according to the present disclosure may comprise at least one of the following other components, if necessary. The other components are not limited; specific examples include water, tracers, ultraviolet fluorescent dyes, stabilizers, and polymerization inhibitors.

When the refrigerant composition according to the present disclosure is used as a working fluid in a refrigerating machine, it is generally used as a mixture with at least a refrigeration oil. Therefore, it is preferable that the refrigerant composition according to the present disclosure does not substantially contain a refrigeration oil. Specifically, in the refrigerant composition according to the present disclosure, the content of the refrigeration oil based on the entire refrigerant composition is preferably 0 to 1 mass%, and more preferably 0 to 0.1 mass%.

The refrigerant composition according to the present disclosure may contain a small amount of water. The water content of the refrigerant composition is preferably 0.1 mass% or less based on the entire refrigerant. A small amount of water contained in the refrigerant composition stabilizes double bonds in the molecules of unsaturated fluorocarbon compounds that can be present in the refrigerant, and makes it less likely that the unsaturated fluorocarbon compounds will be oxidized, thus increasing the stability of the refrigerant composition.

A tracer is added to the refrigerant composition according to the present disclosure at a detectable concentration such that when the refrigerant composition has been diluted, contaminated, or undergone other changes, the tracer can trace the changes.

The refrigerant composition according to the present disclosure may comprise a single tracer, or two or more tracers.

The tracer is not limited, and can be suitably selected from commonly used tracers.

Examples of tracers include hydrofluorocarbons, hydrochlorofluorocarbons, chlorofluorocarbons, hydrochlorocarbons, fluorocarbons, deuterated hydrocarbons, deuterated hydrofluorocarbons, perfluorocarbons, fluoroethers, brominated compounds, iodinated compounds, alcohols, aldehydes, ketones, and nitrous oxide (N₂O). The tracer is particularly preferably a hydrofluorocarbon, a hydrochlorofluorocarbon, a chlorofluorocarbon, a hydrochlorocarbon, a fluorocarbon, or a fluoroether.

The following compounds are preferable as the tracer.
FC-14 (tetrafluoromethane, CF₄)
HCC-40 (chloromethane, CH₃Cl)
HFC-23 (trifluoromethane, CHF₃)
HFC-41 (fluoromethane, CH₃Cl)
HFC-125 (pentafluoroethane, CF₃CHF₂)
HFC-134a (1,1,1,2-tetrafluoroethane, CF₃CH₂F)
HFC-134 (1,1,2,2-tetrafluoroethane, CHF₂CHF₂)
HFC-143a (1,1,1-trifluoroethane, CF₃CH₃)
HFC-152 (1,2-difluoroethane, CH₂FCH₂F)
HFC-245fa (1,1,1,3,3-pentafluoropropane, CF₃CH₂CHF₂)
HFC-236fa (1,1,1,3,3,3-hexafluoropropane, CF₃CH₂CF₃)
HFC-236ea (1,1,1,2,3,3-hexafluoropropane, CF₃CHFCHF₂)
HFC-227ea (1,1,1,2,3,3,3-heptafluoropropane, CF₃CHFCF₃)
HCFC-22 (chlorodifluoromethane, CHClF₂)
HCFC-31 (chlorofluoromethane, CH₂ClF)
CFC-1113 (chlorotrifluoroethylene, CF₂=CClF)
HFE-125 (trifluoromethyl-difluoromethyl ether, CF₃OCHF₂)
HFE-134a (trifluoromethyl-fluoromethyl ether, CF₃OCH₂F)
HFE-143a (trifluoromethyl-methyl ether, CF₃OCH₃)
HFE-227ea (trifluoromethyl-tetrafluoroethyl ether, CF₃OCHFCF₃)
HFE-236fa (trifluoromethyl-trifluoroethyl ether, CF₃OCH₂CF₃)

The refrigerant composition according to the present disclosure may comprise a tracer in a total amount of about 10 parts per million by weight (ppm) to about 1000 ppm. The refrigerant composition according to the present disclosure may comprise a tracer in a total amount of preferably about 30 ppm to about 500 ppm, and more preferably about 50 ppm to about 300 ppm, based on the entire refrigerant composition.

The refrigerant composition according to the present disclosure may comprise a single ultraviolet fluorescent dye, or two or more ultraviolet fluorescent dyes.

The ultraviolet fluorescent dye is not limited, and can be suitably selected from commonly used ultraviolet fluorescent dyes.

Examples of ultraviolet fluorescent dyes include naphthalimide, coumarin, anthracene, phenanthrene, xanthene, thioxanthene, naphthoxanthene, fluorescein, and derivatives thereof. The ultraviolet fluorescent dye is particularly preferably either naphthalimide or coumarin, or both.

The refrigerant composition according to the present disclosure may comprise a single stabilizer, or two or more stabilizers.

The stabilizer is not limited, and can be suitably selected from commonly used stabilizers.

Examples of stabilizers include nitro compounds, ethers, and amines.

Examples of nitro compounds include aliphatic nitro compounds, such as nitromethane and nitroethane; and aromatic nitro compounds, such as nitro benzene and nitro styrene.

Examples of ethers include 1,4-dioxane.

Examples of amines include 2,2,3,3,3-pentafluoropropylamine and diphenylamine.

Examples of stabilizers also include butylhydroxyxylene and benzotriazole.

The content of the stabilizer is not limited. Generally, the content of the stabilizer is preferably 0.01 to 5 mass%, and more preferably 0.05 to 2 mass%, based on the entire mixture of HFC-143 and all of the at least one additional compound.

The refrigerant composition according to the present disclosure may comprise a single polymerization inhibitor, or two or more polymerization inhibitors.

The polymerization inhibitor is not limited, and can be suitably selected from commonly used polymerization inhibitors.

Examples of polymerization inhibitors include 4-methoxy-1-naphthol, hydroquinone, hydroquinone methyl ether, dimethyl-t-butylphenol, 2,6-di-tert-butyl-p-cresol, and benzotriazole.

The content of the polymerization inhibitor is not limited. Generally, the content of the polymerization inhibitor is preferably 0.01 to 5 mass%, and more preferably 0.05 to 2 mass%, based on the entire mixture of HFC-143 and all of the at least one additional compound.

The composition according to the present disclosure can also be used as a refrigeration oil-containing working fluid for a refrigerating machine (this composition being referred to as "the refrigeration oil-containing working fluid according to the present disclosure"). The refrigeration oil-containing working fluid according to the present disclosure comprises at least the refrigerant composition according to the present disclosure and a refrigeration oil, and is used as a working fluid in a refrigerating machine. Specifically, the refrigeration oil-containing working fluid according to the present disclosure is obtained by mixing a refrigeration oil used in a compressor of a refrigerating machine with a mixture of HFC-143 and all of the at least one additional compound or the refrigerant composition. The refrigeration oil-containing working fluid generally comprises 10 to 50 mass% of refrigeration oil.

The refrigeration oil-containing working fluid according to the present disclosure may comprise a single refrigeration oil, or two or more refrigeration oils.

The refrigeration oil is not limited, and can be suitably selected from commonly used refrigeration oils. In this case, refrigeration oils that are superior in the action of increasing the miscibility with the mixture and the stability of the mixture, for example, are suitably selected as necessary.

The base oil of the refrigeration oil is preferably, for example, at least one member selected from the group consisting of polyalkylene glycols (PAG), polyol esters (POE), and polyvinyl ethers (PVE).

The refrigeration oil may further comprise additives in addition to the base oil. The additive may be at least one member selected from the group consisting of antioxidants, extreme-pressure agents, acid scavengers, oxygen scavengers, copper deactivators, rust inhibitors, oil agents, and antifoaming agents.

A refrigeration oil with a kinematic viscosity of 5 to 400 cSt at 40°C is preferable from the standpoint of lubrication.

The refrigeration oil-containing working fluid according to the present disclosure may further optionally comprise at least one additive. Examples of additives include the compatibilizing agents described below.

The refrigeration oil-containing working fluid according to the present disclosure may comprise a single compatibilizing agent, or two or more compatibilizing agents.

The compatibilizing agent is not limited, and can be suitably selected from commonly used compatibilizing agents.

Examples of compatibilizing agents include polyoxyalkylene glycol ethers, amides, nitriles, ketones, chlorocarbons, esters, lactones, aryl ethers, fluoroethers, and 1,1,1-trifluoroalkanes. The compatibilizing agent is particularly preferably a polyoxyalkylene glycol ether.

### 2. Separation Method

The present disclosure also discloses a method for separating the components by using the above composition.

For example, an additional component can be separated from HFC-143 by extracting an azeotropic or azeotrope-like composition containing HFC-143 and the additional component from a composition containing at least HFC-143 and the additional component by azeotropic distillation.

More specifically, the separation method according to the present disclosure may be a method for separating an additional compound from HFC-143 in a mixture containing HFC-143 and at least one additional compound,
the method comprising
(1) supplying a starting composition containing HFC-143 and the additional compound to a first distillation column to extract
   an azeotropic mixture composition containing HFC-143 and the additional compound as a first distillate, and
   a composition that is richer in concentration of either HFC-143 or the additional compound than the starting composition as a first distillation-column-bottom composition; and
(2) optionally supplying the first distillate or the first distillation-column-bottom composition to a second distillation column that is different from the first distillation column in terms of operation conditions, to form a composition that is richer in concentration of either HFC-143 or the additional compound than the starting composition.

In the method above, the additional compound is, for example, at least one member selected from the group consisting of 1,1-difluoroethylene (HFO-1132a), 1,1,2-trifluoroethylene (HFO-1123), 1,1,1-trifluoromethane (HFC-143a), 1-chloro-1,2,2-trifluoroethylene (CTFE), 1-chloro-1-fluoroethylene (HCFO-1131a), 1,1-difluoroethane (HFC-152a), 1,2-difluoroethane (HFC-152), trans-1-chloro-2-fluoroethylene (HCFO-1131(E)), cis-1-chloro-2-fluoroethylene (HCFO-1131(Z)), 1,1,1,2-tetrafluoroethane (HFC-134a), fluoroethane (HFC-161), 1,2-dichloro-1-fluoroethane (HCFC-141), 1,1-dichloro-2,2,2-trifluoroethane (HCFC-123), 1-chloro-2,2-difluoroethane (HCFC-142), 1-chloro-1,2-difluoroethane (HCFC-142a), 1-chloro-2,2-difluoroethylene (HCFO-1122), 1-chloro-1,2-difluoroethylene (HCFO-1122a), fluoroethylene (HFO-1141), dichlorofluoromethane (HCFC-21), chlorodifluoromethane (HCFC-22), trifluoromethane (HFC-23), ethylene, and acetylene.

In the steps above, the method and conditions of supply to each distillation column, as well as the specifications of each distillation column and the conditions of distillation, can be suitably set to achieve the purpose of each step.

The embodiments are described above; however, it will be understood that various changes in forms and details can be made without departing from the spirit and scope of the claims.

### Examples

The present disclosure is described in more detail below with reference to Examples. However, the present disclosure is not limited to the Examples.

### Example 1

Table 1 shows the calculation results of the refrigeration cycle of compositions containing HFC-143 and various additional compounds. The calculation conditions are as follows: evaporation temperature: 10°C, condensation temperature: 45°C, degree of superheat: 5°C, degree of supercooling: 5°C, compression efficiency: 70%.

Peng-Robinson was used as a physical properties model.

**Table 1**

| Component | Formulation (comparison by mass) | COP | Refrigerating Capacity |
|---|---|---|---|
| | | (HFC-245fa: relative to conventional refrigerant) | |
| HFC-143/HFO-1132a | 99/1 | 1.010 | 1.655 |
| HFC-143/HFO-1123 | 99/1 | 1.014 | 1.648 |
| HFC-143/HFC-143a | 99/1 | 1.015 | 1.645 |
| HFC-143/CTFE | 99/1 | 1.016 | 1.636 |
| HFC-143/HCFO-1131a | 99/1 | 1.017 | 1.639 |
| HFC-143/HCFO-1131(E) | 99/1 | 1.017 | 1.634 |
| HFC-143/HCFO-1131(Z) | 99/1 | 1.017 | 1.628 |
| HFC-1431HFC-152 | 99/1 | 1.016 | 1.645 |
| HFC-143/HFC-152a | 99/1 | 1.016 | 1.643 |
| HFC-143/HFC-134a | 99/1 | 1.016 | 1.638 |
| HFC-143/HFC-161 | 99/1 | 1.015 | 1.657 |
| HFC-143/HCFC-141 | 99/1 | 1.020 | 1.612 |
| HFC-143/HCFC-123 | 99/1 | 1.017 | 1.619 |
| HFC-143/HCFC-142 | 99/1 | 1.018 | 1.617 |
| HFC-143/HCFC-142a | 99/1 | 1.017 | 1.624 |
| HFC-143/HCFO-1122 | 99/1 | 1.017 | 1.636 |
| HFC-143/HCFO-1122a | 99/1 | 1.017 | 1.629 |
| HFC-143/HFO-1141 | 99/1 | 1.010 | 1.666 |
| HFC-143/HCFC-21 | 99/1 | 1.017 | 1.625 |
| HFC-143/HFO-22 | 99/1 | 1.016 | 1.644 |
| HFC-143/HFO-23 | 99/1 | 1.011 | 1.654 |
| HFC-143/Ethylene | 99/1 | 0.923 | 1.566 |
| HFC-143/Propylene | 99/1 | 0.944 | 1.605 |
| HFC-143/Acetylene | 99/1 | 1.013 | 1.663 |

### Example 2

### Method for Producing HFC-143

A 50A reaction tube was prepared. This reaction tube was filled with 670 g of a catalyst. The catalyst was formed with activated carbon as a support and palladium as a noble metal. CTFE (chlorotrifluoroethylene) at a flow rate of 500 Nml/min and hydrogen at a flow rate of 1800 Nml/min were supplied from the supply port of the reaction tube filled with the catalyst, and the catalyst was passed through the upstream section and then the downstream section. At this stage, the temperature of the upstream section was set to 320°C, and the temperature of the downstream section was set to 230°C. In the above reaction, the contact time expressed by W/Fo was 17.5 g·sec/cc: W (g) is the volume of the catalyst filled in the reaction tube, and Fo is the total flow rate of fluoroethylene and hydrogen gas into the reaction tube. Table 2 shows the results.

**Table 2**

| Detected Component | Detection Amount (mol%) |
|---|---|
| | Example 1 |
| HFC-143 | 93.0 |
| HFO-1123 | 0.9 |
| HCFC-133b | 1.2 |
| HCFC-133 | 0.8 |
| CTFE | 0.0 |

HFC-143, a target product, can be obtained at a high yield.

### Example 3

A compositions containing HFC-143 and HFO-1123, ethylene, HFC-161, HFC-152, HCFC-133, HCFC-123, and HCFC-21 as the additional component was distilled by using the distillation columns shown in Fig. 1. Table 3 shows the results.

**Table 3**

| | Flow Rate (kg/hr) | | | | |
|---|---|---|---|---|---|
| | S11 | S12 | S13 | S14 S15 | |
| HFC143 | 0.92 | 0.9 | 0.02 | 0.00 | 0.90 |
| HFO-1123 | 0.02 | 0.00 | 0.02 | 0.00 | 0.00 |
| Ethylene | 0.01 | 0.00 | 0.01 | 0.00 | 0.00 |
| HFC-161 | 0.01 | 0.00 | 0.01 | 0.00 | 0.00 |
| HFC-152 | 0.01 | 0.01 | 0.00 | 0.01 | 0.00 |
| HCFC-133 | 0.01 | 0.01 | 0.00 | 0.01 | 0.00 |
| HCFC-123 | 0.01 | 0.01 | 0.00 | 0.01 | 0.00 |
| HCFC-21 | 0.01 | 0.01 | 0.00 | 0.01 | 0.00 |

As described above, high-purity HFC-143 was obtained by separating the additional component by distillation using an azeotrope-like composition containing HFC-143 and HFO-1123, ethylene, and HFC-161.

## Claims

1. A composition comprising
1,1,2-trifluoroethane (HFC-143), and
at least one additional compound selected from the group consisting of 1,1-difluoroethylene (HFO-1132a), 1,1,2-trifluoroethylene (HFO-1123), 1,1,1-trifluoromethane (HFC-143a), 1-chloro-1,2,2-trifluoroethylene (CTFE), 1-chloro-1-fluoroethylene (HCFO-1131a), 1,1-difluoroethane (HFC-152a), 1,2-difluoroethane (HFC-152), trans-1-chloro-2-fluoroethylene (HCFO-1131(E)), cis-1-chloro-2-fluoroethylene (HCFO-1131(Z)), 1,1,1,2-tetrafluoroethane (HFC-134a), fluoroethane (HFC-161), 1,2-dichloro-1-fluoroethane (HCFC-141), 1,1-dichloro-2,2,2-trifluoroethane (HCFC-123), 1-chloro-2,2-difluoroethane (HCFC-142), 1-chloro-1,2-difluoroethane (HCFC-142a), 1-chloro-2,2-difluoroethylene (HCFO-1122), 1-chloro-1,2-difluoroethylene (HCFO-1122a), fluoroethylene (HFO-1141), dichlorofluoromethane (HCFC-21), chlorodifluoromethane (HCFC-22), trifluoromethane (HFC-23), ethylene, and acetylene.

2. The composition according to claim 1, wherein HFC-143 and the additional compound are in the form of an azeotropic or azeotrope-like mixture thereof.

3. The composition according to claim 1 or 2, wherein the additional compound is present in a total amount of more than 0 mass% and 30 mass% or less, based on the entire mixture of HFC-143 and the additional compound.

4. Use of the composition of any one of claims 1 to 3 as a heat transfer medium composition.

5. A method for separating an additional compound from HFC-143 in a mixture containing HFC-143 and the additional compound,
the additional compound being at least one compound selected from the group consisting of 1,1-difluoroethylene (HFO-1132a), 1,1,2-trifluoroethylene (HFO-1123), 1,1,1-trifluoromethane (HFC-143a), 1-chloro-1,2,2-trifluoroethylene (CTFE), 1-chloro-1-fluoroethylene (HCFO-1131a), 1,1-difluoroethane (HFC-152a), 1,2-difluoroethane (HFC-152), trans-1-chloro-2-fluoroethylene (HCFO-1131(E)), cis-1-chloro-2-fluoroethylene (HCFO-1131(Z)), 1,1,1,2-tetrafluoroethane (HFC-134a), fluoroethane (HFC-161), 1,2-dichloro-1-fluoroethane (HCFC-141), 1,1-dichloro-2,2,2-trifluoroethane (HCFC-123), 1-chloro-2,2-difluoroethane (HCFC-142), 1-chloro-1,2-difluoroethane (HCFC-142a), 1-chloro-2,2-difluoroethylene (HCFO-1122), 1-chloro-1,2-difluoroethylene (HCFO-1122a), fluoroethylene (HFO-1141), dichlorofluoromethane (HCFC-21), chlorodifluoromethane (HCFC-22), trifluoromethane (HFC-23), ethylene, and acetylene,
the method comprising
(1) supplying a starting composition containing HFC-143 and the additional compound to a first distillation column to extract
an azeotropic or azeotrope-like mixture composition containing HFC-143 and the additional compound as a first distillate, and
a composition that is richer in concentration of either HFC-143 or the additional compound than the starting composition as a first distillation-column-bottom composition; and
(2) optionally supplying the first distillate or the first distillation-column-bottom composition to a second distillation column that is different from the first distillation column in terms of operation conditions to form a composition that is richer in concentration of either HFC-143 or the additional compound than the starting composition.
